(19) Europäisches Patentamt
European Patent Office
Office européen des brevets

(11) Veröffentlichungsnummer: **0 025 882**
**A1**

(12) # EUROPÄISCHE PATENTANMELDUNG

(21) Anmeldenummer: 80104975.0

(22) Anmeldetag: 21.08.80

(51) Int. Cl.³: C 07 D 249/08
C 07 D 233/56, C 07 D 233/58
A 01 N 43/64, A 01 N 43/50

(30) Priorität: 01.09.79 DE 2935452

(43) Veröffentlichungstag der Anmeldung:
01.04.81 Patentblatt 81/13

(84) Benannte Vertragsstaaten:
AT BE CH DE FR GB IT LI LU NL SE

(71) Anmelder: BASF Aktiengesellschaft
Carl-Bosch-Strasse 38
D-6700 Ludwigshafen(DE)

(72) Erfinder: Buschmann, Ernst, Dr.
Georg-Ludwig-Krebs-Strasse 10
D-6700 Ludwigshafen(DE)

(72) Erfinder: Zeeh, Bernd, Dr.
Thorwaldsenstrasse 5
D-6700 Ludwigshafen(DE)

(72) Erfinder: Pommer, Ernst-Heinrich, Dr.
Berliner Platz 7
D-6703 Limburgerhof(DE)

(54) **N-phenylpropylsubstituierte Azole, Verfahren zu ihrer Herstellung und diese enthaltende Fungizide.**

(57) N-Phenylpropylsubstituierte Azole der Formel

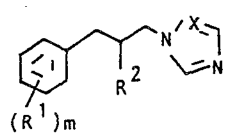

in der $R^1$ einen Alkylrest oder Halogen, $R^2$ Wasserstoff, einen Alkylrest oder Aralkylrest, der durch Halogen substituiert sein kann, m die Zahlen 0 bis 3 und X CH oder N bedeuten, sowie deren pflanzenphysiologisch verträgliche Säureadditionssalze und Metallkomplexe, Verfahren zu ihrer Herstellung und diese enthaltende Fungizide.

EP 0 025 882 A1

0025882

BASF Aktiengesellschaft O.Z. 0050/034027

N-phenylpropylsubstituierte Azole, Verfahren zu ihrer Herstellung und diese enthaltende Fungizide

Die vorliegende Erfindung betrifft neue wertvolle N-phenylpropylsubstituierte Azole sowie ihre Salze und Metallkomplexe und Fungizide, die diese Verbindungen enthalten.

Es ist bekannt, N-phenylsubstituierte Morpholine, z.B. das N-[3-(p-tert.-Butylphenyl)-2-methyl-propyl]-cis-2,6-dimethylmorpholin als Wirkstoff eines Fungizids zu verwenden. (DE-OS 2 656 747). Seine Wirkung befriedigt jedoch nicht.

Es wurde gefunden, daß die neuen N-phenylpropylsubstituierten Azole der Formel

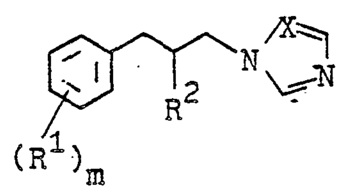

I

in der

$R^1$ einen Alkylrest oder Halogen,

$R^2$ Wasserstoff, einen Alkylrest oder Aralkylrest, der durch Halogen substituiert sein kann,

m die Zahlen 0 bis 3 und

X CH oder N bedeuten sowie deren pflanzenphysiologisch verträgliche Säureadditionssalze und Metallkomplexe eine überraschend gute fungizide Wirksamkeit zeigen.

Sws/BL

$(R^1)_m$ bedeutet beispielsweise Methyl, Chlor, Brom z.B. als spezielle Substituenten am Phenylrest 4-Chlor, 2,4-Dichlor, 2,3,4-Trichlor, 3,4-Dichlor, 2,4,5-Trichlor, 2-Chlor, 4-Brom, 2-Methyl-4-Chlor,

$R^2$ bedeutet beispielsweise Wasserstoff, Methyl, Ethyl, Propyl, Isopropyl, n-Butyl, tert.-Butyl, n-Pentyl, n-Hexyl, Benzyl, 4-Chlorbenzyl, 2,4-Dichlorbenzyl, 3,4-Dichlorbenzyl, 4-Brombenzyl, 4-Methylbenzyl.

Typische pflanzenphysiologisch verträgliche Säureadditionssalze der neuen Phenylpropylazole sind Chloride, Bromide, Jodide, Fluoride, Sulfate, Nitrate, Phosphate, Acetate, Citrate, Oxalate, Tartrate, Malate (Salze der Äpfelsäure) und Maleate.

Die neuen Phenylpropylazole werden hergestellt, indem man Verbindungen der Formel

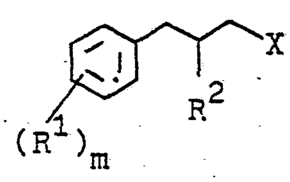

II

in der
$R^1$, $R^2$ und m die obengenannten Bedeutungen haben und X für Halogen (Chlor, Brom, Jod) oder eine andere nucleofuge Gruppe wie $OSO_3R$ (R = Alkyl), p-Methyl-benzolsulfonyl, p-Methoxybenzolsulfonyl, steht, umsetzt mit 1,2,4-Triazol oder mit Imidazol.

Die Umsetzung wird beispielsweise in höhersiedenden organischen Lösungsmitteln oder ohne Lösungsmittel im Temperaturbereich von 80 bis 200°C unter eventuellem Zusatz eines

Säurefängers bei Normaldruck oder erhöhtem Druck durchgeführt.

Geeignete Lösungsmittel sind beispielsweise Äthanol, Methanol, Acetonitril, Dimethylformamid, Dimethylacetamid, Dimethylsulfoxid, Hexamethylphosphorsäuretriamid, N-Methylpyrolidon.

Geeignete Säurefänger sind beispielsweise $NaOCH_3$, $NaOC_2H_5$, NaH, $Na_2CO_3$, $K_2CO_3$. Als Säurefänger kann auch überschüssiges Triazol oder Imidazol dienen.

Bevorzugt wird die Umsetzung ohne Lösungsmittel oder in Acetonitril oder Dimethylformamid bei 80 bis 160°C bei Normaldruck durchgeführt.

Die Ausgangsverbindunen der Formel II werden nach allgemein bekannten Reaktionen nach folgendem Schema hergestellt: (wobei $R^1$ und $R^2$ die oben genannten Bedeutungen haben).

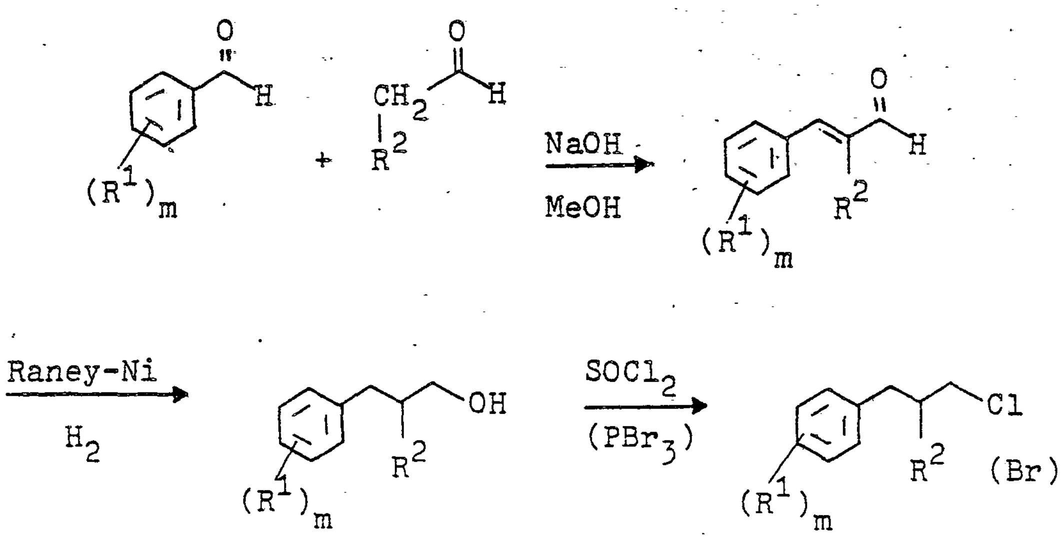


Die folgenden Beispiele erläutern die Herstellung der neuen Verbindungen:

1. N-[3-(4-Chlorphenyl)-2-isopropyl-propyl]-imidazol

Ein Gemisch von 33 g 1-Brom-3-(4-Chlorphenyl)-2-isopropyl-propan und 25 g Imidazol wird 5 h (Stunden) auf 150°C erwärmt. Nach dem Abkühlen wird in Chloroform gelöst, mit verdünnter wäßriger NaOH und anschließend mit Wasser gewaschen, über $Na_2SO_4$ getrocknet und eingedampft. Die Destillation des Rohproduktes ergibt 23,5 g N-[3-(4-Chlorphenyl)-2-isopropyl-propyl]-imidazol.
Sdp. 152 bis 156°C, 0,1 mbar. (Wirkstoff Nr. 10)

2. 1-[3-(4-Chlorphenyl)-2-isopropyl-propyl]-1,2,4-triazol

Eine Lösung von 50 g 1-Brom-3-(4-chlorphenyl)-2-isopropyl-propan und 32 g 1,2,4-Triazol in 300 ml Dimethylformamid wird 5 h zum Rückfluß erwärmt. Man läßt abkühlen und destilliert das Lösungsmittel im Vakuum einer Wasserstrahlpumpe ab. Der Rückstand wird in Chloroform gelöst, mit verdünnter wäßriger NaOH und anschließend mit Wasser gewaschen, über $Na_2SO_4$ getrocknet und eingedampft. Die Destillation des Rohproduktes ergibt 25 g 1-[3-(4-Chlorphenyl)-2-isopropyl-propyl]-1,2,4-triazol.
Sdp. 150 bis 170°C/0,1 mbar (Wirkstoff Nr. 45).

Das destillierte Produkt wird in Essigester gelöst. Bei 10°C wird konz. $HNO_3$ zugetropft. Das auskristallisierte Produkt wird abgesaugt, mit Essigester gewaschen und im Vakuum bei 50°C getrocknet. Man erhält 20 g 1-[3-(4-Chlorphenyl)-2-isopropyl-propyl]-1,2,4-Triazol . $HNO_3$. (Wirkstoff Nr. 46).

3. N-[3-(4-Bromphenyl)-propyl]-imidazol

Ein Gemisch von 70 g 3-(4-Bromphenyl)-1-chlor-propan und 61 g Imidazol wird 5 h auf 150°C erwärmt. Nach dem Abkühlen wird in Chloroform gelöst, mit verdünnter wäßriger NaOH und anschließend mit Wasser gewaschen, über $Na_2SO_4$ getrocknet und eingedampft. Die Destillation des Rohproduktes ergibt 50 g N-[3-(4--Bromphenyl)]-propyl-imidazol, Sdp. 176°C 0,2 mbar. (Wirkstoff Nr. 4).

4. 1-[3-(2,4-Dichlorphenyl)-propyl-1,2,4-triazol-1/2$CuCl_2$

Eine Lösung von 46 g 1-[3-(2,4-Dichlorphenyl)-propyl--1,2,4-triazol und 26,4 g $CuCl_2.2H_2O$ in 300 ml Ethanol wird 10 min zum Rückfluß erwärmt. Man läßt abkühlen und gibt 500 ml Wasser zu. Dabei fällt das Produkt als Öl aus. Man dekantiert ab und verreibt das Produkt mit Ether. Dabei kristallisiert der gewünschte Kupferkomplex aus.
Ausbeute 30 g, hellgrüne Kristalle, Schmp. 132°Zers.

In entsprechender Weise erhält man die folgenden Wirkstoffe:

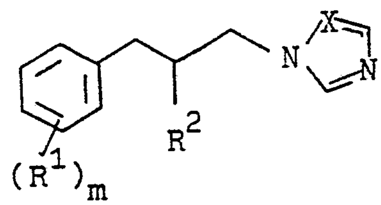 · HY

| Wirkstoff Nr. | $(R^1)_m$ | $R^2$ | X | Y | Siedepunkt ($^{o}C$) | Schmelzpunkt $^{o}C$ |
|---|---|---|---|---|---|---|
| 1 | 4-Br | H | N | $NO_3$ | | $132^{o}C$ (Zersetzg.) |
| 2 | 4-Br | n-Butyl | N | $NO_3$ | | |
| 3 | 4-Br | Benzyl | N | $NO_3$ | | |
| 4 | 4-Br | H | CH | | $176^{o}$ 0,2mbar | |
| 5 | 4-Br | n-Butyl | CH | | | |
| 6 | 4-Cl | n-Propyl | N | $NO_3$ | | $130^{o}C$ |
| 7 | 4-Cl | n-Butyl | N | $NO_3$ | | $116^{o}C$ |
| 8 | 4-Cl | n-Hexyl | N | | $170^{o}$ 0,1mbar | |
| 9 | 4-Cl | n-Butyl | CH | | | |
| 10 | 4-Cl | Isopropyl | CH | | $152-156^{o}$ 0,1mbar | |
| 11 | 4-Cl | n-Propyl | CH | | $171-6^{o}$0,1mbar | |
| 12 | 4-Cl | Benzyl | N | $NO_3$ | | |
| 13 | 2,4-Cl | $CH_3$ | N | $NO_3$ | | $142^{o}C$ |
| 14 | 2,4-Cl | $CH_3$ | N | | $160-70^{o}$ 0,1mbar | |
| 15 | 2,4-Cl | $C_2H_5$ | N | $NO_3$ | | |
| 16 | 2,4-Cl | $C_2H_5$ | N | | | |
| 17 | 2,4-Cl | Isopropyl | CH | | $160^{o}$ 0,1mbar | |
| 18 | 2,4-Cl | Isopropyl | N | $NO_3$ | | $130^{o}C$ |
| 19 | 2,4-Cl | Isopropyl | N | | | |

| Wirkstoff Nr. | $(R^1)_m$ | $R^2$ | X | Y | Siedepunkt (°C) | Schmelzpunkt °C |
|---|---|---|---|---|---|---|
| 20 | 2,4-Cl | n-Propyl | N | $NO_3$ | | 100°C |
| 21 | 2,4-Cl | n-Propyl | N | | | |
| 22 | 2,4-Cl | n-Propyl | CH | | | |
| 23 | 2,4-Cl | n-Butyl | N | $NO_3$ | | 106°C |
| 24 | 2,4-Cl | n-Butyl | N | | | Öl |
| 25 | 2,4-Cl | n-Butyl | CH | | 200-204° 0,2mbar | |
| 26 | 2,4-Cl | t-Butyl | N | $NO_3$ | | |
| 27 | 2,4-Cl | t-Butyl | CH | | | |
| 28 | 2,4-Cl | n-Pentyl | N | $NO_3$ | | 109°C |
| 29 | 2,4-Cl | n-Pentyl | N | | | Öl |
| 30 | 2,4-Cl | n-Hexyl | N | - | | |
| 31 | 2,4-Cl | n-Hexyl | N | $NO_3$ | | |
| 32 | 2,4-Cl | Benzyl | N | $NO_3$ | | 130°Zers. |
| 33 | 2,4-Cl | Benzyl | CH | | | |
| 34 | 2,4-Cl | 4-Cl-Benzyl | N | $NO_3$ | | |
| 35 | 3,4-Cl | n-Butyl | N | $NO_3$ | | |
| 36 | 3,4-Cl | Benzyl | N | $NO_3$ | | |
| 37 | 2,3,4-Cl | n-Butyl | N | $NO_3$ | | |
| 38 | 2,4,5-Cl | H | N | $NO_3$ | | 140°C |
| 39 | 2,4,5-Cl | n-Butyl | N | | | |
| 40 | - | n-Butyl | N | | 130-140° 0,1mbar | |
| 41 | - | n-Butyl | N | $NO_3$ | | 78° |
| 42 | $2-CH_3-4-Cl$ | n-Butyl | N | $NO_3$ | | |

| Wirkstoff Nr. | $(R^1)_m$ | $R^2$ | X | Y | Siedepunkt (°C) | Schmelzpunkt °C |
|---|---|---|---|---|---|---|
| 43 | 2-$CH_3$-4-Cl | n-Butyl | N | | | |
| 44 | 2-Cl | n-Butyl | N | $NO_3$ | | |
| 45 | 4-Cl | Isopropyl | N | | 150-170° 0,1mbar | |
| 46 | 4-Cl | Isopropyl | N | $NO_3$ | | |
| 47 | 2,3,4-Cl | n-Butyl | N | | | |
| 48 | 2,3,4-Cl | n-Butyl | N | $NO_3$ | | |
| 49 | 2,4-Cl | n-Propyl | N | Cu-Komplex | | 132° |
| 50 | 2,4-Cl | iso-Propyl | N | Cu-Komplex | | 120° |

Die neuen Verbindungen und ihre Salze und Metallkomplexverbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden. Ferner können sie auch im Materialschutz (z.B. Holzschutz) verwendet werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, sowie Gemüse - wie Gurken, Bohnen und Kürbisgewächse - .

Die neuen Verbindungen sind insbesondere geeignet zur Bekämpfung folgender Pflanzenkrankheiten:

Erysiphe graminis (echter Mehltau) in Getreide,
Erysiphe cichoriacearum (echter Mehltau) an Kürbisgewächsen,
Podosphaera leucotricha an Äpfeln,
Uncinula necator an Reben,
Erysiphe polygoni an Bohnen,
Sphaerotheca pannosa an Rosen,
Puccinia-Arten an Getreide,
Rhizoctonia solani an Baumwolle sowie
Helmingthosporiumarten an Getreide,
Ustilgago-Arten an Getreide und Zuckerrohr,
Rhynchosporium secale an Getreide,
Venturia inaequalis (Apfelschorf).

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung oder wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle, z.B. Kaoline, Tonerden, Talkum, Kreide und synthetische Gesteinsmehle (z.B. hochdisperse Kieselsäure, Silikate); Emulgiermittel, wie nichtionogene und anionische Emulgatoren (z.B. Polyoxyethylen - Fettalkohole - Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,1 und 3 kg Wirkstoff oder mehr je ha. Die neuen Verbindungen können auch im Materialschutz u.a. zur Bekämpfung holzzerstörender Pilze, wie Coniophora puteana und Polystictus versicolor eingesetzt werden. Bei der Anwendung der Wirkstoffe im Materialschutz, z.B. als Fungizide für Anstrichfarben und Weich-Polyvinylchlorid, betragen die Aufwandmengen 0,05 bis 5 Gew.-% Wirkstoff, bezogen auf das Gesamtgewicht der zu konservierenden Farben bzw. des mikrozidauszurüstenden Polyvinylchlorids. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung des Beispiels 1 mit 10 Gewichtsteilen N-Methyl- -pyrrolidon und erhält eine Lösung, die zur Anwendung in Form kleinster Tropfen geeignet ist.

II. 20 Gewichtsteile der Verbindung des Beispiels 3 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.

III. 20 Gewichtsteile der Verbindung des Beispiels 3 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

IV. 20 Gewichtsteile der Verbindung des Beispiels 1 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Ge-

wichtsteilen Wasser erhält man eine wäßrige Dispersion, die 0,02 Gew.-% des Wirkstoffs enthält.

V. 20 Gewichtsteile der Verbindung des Beispiels 3 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.

VI. 3 Gewichtsteile der Verbindung des Beispiels 1 werden mit 97 Gewichtsteilen feinteiligem Kaolin innig vermischt. Man erhält auf diese Weise ein Stäubemittel, das 3 Gew.-% des Wirkstoffs enthält.

VII. 30 Gewichtsteile der Verbindung des Beispiels 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung des Beispiels 3 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,0 Gew.-% Wirkstoff enthält.

IX. 20 Teile der Verbindung des Beispiels 1 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie Ferridimethyldithiocarbamat, Zinkdimethyldithiocarbamat, Manganethylenbisdithiocarbamat, Mangan-Zink-ethylendiamin-bis-dithiocarbamat oder Zinkethylenbisdithiocarbamat,
Tetramethylthiuramidsulfide,
Ammoniak-Komplex von Zink-(N,N-ethylen-bis-dithiocarbamat) und N,N'-Polyethylen-bis-(thiocarbamoyl)-disulfid,
Zink-(N,N'-propylen-bis-dithiocarbamt),
Ammoniak-Komplex von Zink-(N,N'-propylen-bis-dithiocarbamat) und N,N'-Propylen-bis(thiocarbamoyl)-disulfid;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
heterocyclische Substanzen, wie
N-Trichlormethylthio-tetrahydrophthalimid,
N-(1,1,2,2-Tetrachlorethylthio)-tetrahydrophthalimid,
N-Trichlormethylthio-phthalimid,
2-Heptadecyl-2-imidazolin-acetat,
2,4-Dichlor-6-(o-chloranilino)-s-triazin,
O,O-Diethyl-phthalimidophosphonothioat,
5-Amino-1-(bis-(dimethylamino)-phosphinyl)-3-phenyl-1,2,4-triazol,
5-Ethoxy-3-trichlormethyl-1,2,4-thiadiazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-(2))-benzimidazol,
Piperazin-1,4-diyl-bis-(1-(2,2,2-trichlor-ethyl)-formamid),
2-(Thiazolyl-(4))-benzimidazol,
5-Butyl-2-dimethylamino-4-hydroxy-6-methyl-pyrimidin,
Bis-(p-Chlorphenyl)-pyridinmethanol,
1,2-Bis-(3-ethoxycarbonyl-2-thioureido)-benzol,
1,2-Bis-(3-methoxycarbonyl-2-thioureido)-benzol

und weitere Substanzen wie
Dodecylguanidinacetat,
3-(3-(3,5-Dimethyl-2-oxycyclohexyl)-2-hydroxyethyl)-glutarimid,
Hexachlorbenzol,
N-Dichlorfluormethylthio-N',N'-dimethyl-N-phenyl-schwefelsäurediamid,
2,5-Dimethyl-furan-3-carbonsäureanilid,
2,5-Dimethyl-furan-3-carbonsäure-cyclohexylamid,
2-Cyan-N-(ethylaminocarbonyl)-2-(methoxyimino)-acetamid,
2-Methyl-benzoesäure-anilid,
2-Jod-Benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin b1w. dessen Salze,

DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
5-Nitro-isophthalsäure-di-isopropylester,
1-(1',2',4'-Triazolyl-1')-(4'-chlorphenoxy)-3,3-dimethylbutan-2-on,
1-(1',2',4'-Triazolyl-1')-1-(4'-chlorphenoxy)-3,3-dimethylbutan-2-ol,
N-(2,6-Dimethylphenyl)-N-chloracetyl-D,L-2-aminobutyrolacton,
N-(n-Propyl)-N-(2,4,6-trichlorphenoxyethyl)-N'-imidazolylharnstoff.

Die folgenden Beispiele zeigen die biologische Wirkung der neuen Substanzen. Als Vergleichssubstanz diente das aus der DE-OS 26 56 747 bekannte N-[3-(p-tert.-Butylphenyl)-2-methyl-propyl]-cis-2,6-dimethylmorpholin (Wirkstoff A).

Beispiel A

Wirksamkeit gegen Weizenmehltau

Blätter von in Töpfen gewachsenen Weizenkeimlingen der Sorte "Jubilar" werden mit wäßrigen Emulsionen aus 80 % (Gewichtsprozent) Wirkstoff und 20 % Emulgiermittel besprüht und nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80 % relativer Luftfeuchtigkeit aufgestellt. Nach 10 Tagen wird das Ausmaß der Mehltaupilzentwicklung ermittelt.

| Wirkstoff | Befall der Blätter nach Spritzungen mit x%iger Wirkstoffbrühe x = 0,025 | 0,012 | 0,006 |
|---|---|---|---|
| 4 | 0 | 0 | 0 |
| 8 | 0 | 0 | 1 |
| 11 | 0 | 0 | 0 |
| 20 | 0 | 0 | 0 |
| 23 | 0 | 0 | 0 |
| 24 | 0 | 1 | 1 |
| 25 | 0 | 0 | 0 |
| 28 | 0 | 0 | 0 |
| 29 | 0 | 0 | 0 |
| 35 | 0 | 0 | 0 |
| 38 | 0 | 0 | 0 |
| 40 | 0 | 0 | 2-3 |
| 41 | 0 | 0 | 2 |
| Kontrolle (unbehandelt) | 5 | | |

0 = kein Pilzbefall, abgestuft bis 5 - Totalbefall

Beispiel B

Wirksamkeit gegen Weizenbraunrost

Blätter von in Töpfen gewachsenen Weizenpflanzen werden mit Sporen des Weizenbraunrostes (Puccinia recondita) künstlich infiziert und 48 Stunden lang bei 20 bis 25°C in einer wasserdampfgesättigten Kammer aufgestellt. Danach werden die Pflanzen mit wäßrigen Spritzbrühen, die in dem Wasser gelöst oder emulgiert eine Mischung aus 80 % des zu prüfenden Wirkstoffes und 20 % Natriumligninsulfonat enthalten, besprüht und im Gewächshaus bei Temperaturen zwischen 20 und 22°C und bei 75 bis 80 % relativer Luftfeuchtigkeit aufgestellt. Nach 10 Tagen wird das Ausmaß der Rostpilzentwicklung beurteilt.

| Wirkstoff Nr. | Befall der Blätter nach Spritzungen mit x%iger Wirkstoffbrühe x = 0,025 | 0,012 | 0,006 |
|---|---|---|---|
| 20 | 0 | 1 | 2 |
| 23 | 0 | 0 | 0 |
| 25 | 0 | 1 | 2 |
| 29 | 0 | 0 | 4 |
| Kontrolle (unbehandelt) | 5 | | |

0 = kein Pilzbefall, abgestuft bis 5 - Totalbefall

Beispiel C

Wirksamkeit gegen Gurkenmehltau

Blätter von in Töpfen gewachsenen Gurkenkeimlingen der Sorte "Chinesische Schlangen" werden im Zweiblattstadium mit wäßrigen Emulsionen aus 80 % Wirkstoff und 20 % Emulgiermittel besprüht und nach dem Antrocknen des Spritzbelages mit Oidien (Sporen) des Gurkenmehltaus (Erysiphe cichoriacearum) bestäubt. Die Versuchspflanzen werden anschließend im Gewächshaus bei Temperaturen zwischen 20 und 22°C und 70 bis 80 % relativer Luftfeuchtigkeit aufgestellt. Zur Beurteilung der Wirkungsdauer der neuen Wirkstoffe wird das Ausmaß der Mehltaupilzentwicklung nach 21 Tagen ermittelt.

| Wirkstoff Nr. | Befall der Blätter nach Spritzung mit 0,025%iger Wirkstoffbrühe |
|---|---|
| 4 | 1 |
| 6 | 1 + |
| 8 | 0 |
| 20 | 0 |
| 24 | 0 |
| 25 | 1 |
| 28 | 0 |
| 29 | 0 |
| 35 | 0 |
| 38 | 1 + |
| A (Vergleichsmittel) | 3 |
| Kontrolle (unbehandelt) | 5 |

+) leichte Blattschädigungen

0 = kein Pilzbefall, abgestuft bis 5 - Totalbefall

Patentansprüche

1. N-Phenylpropylsubstituiertes Azol der Formel

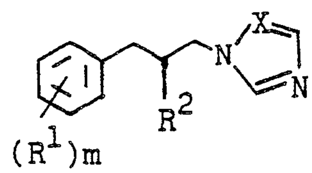

in der $R^1$ einen Alkylrest oder Halogen, $R^2$ Wasserstoff, einen Alkylrest oder Aralkylrest, der durch Halogen substituiert sein kann, m die Zahlen 0 bis 3 und X CH oder N bedeuten, sowie deren pflanzenphysiologisch verträgliche Säureadditionssalze und Metallkomplexe.

2. Fungizid enthaltend N-Phenylpropylsubstituiertes Azol der Formel

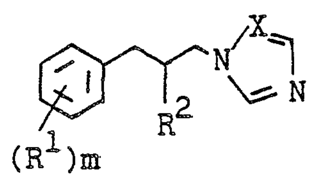

in der $R^1$ einen Alkylrest oder Halogen, $R^2$ Wasserstoff, einen Alkylrest oder Aralkylrest, der durch Halogen substituiert sein kann, m die Zahlen 0 bis 3 und X CH oder N bedeuten, sowie deren pflanzenphysiologisch verträgliche Säureadditionssalze und Metallkomplexe.

3. Fungizid enthaltend einen festen oder flüssigen Trägerstoff und ein N-Phenylpropylsubstituiertes Azol der Formel

346/79 Sws/Br

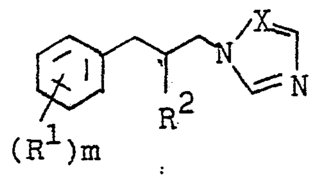

in der $R^1$ einen Alkylrest oder Halogen,
$R^2$ Wasserstoff, einen Alkylrest oder Aralkylrest, der durch Halogen substituiert sein kann,
m die Zahlen 0 bis 3 und
X CH oder N bedeuten,
sowie deren pflanzenphysiologisch verträgliche Säureadditionssalze und Metallkomplexe.

4. Verfahren zur Herstellung eines Fungizids, dadurch gekennzeichnet, daß man einen festen oder flüssigen Trägerstoff vermischt mit einem N-Phenylpropylsubstituierten Azol der Formel

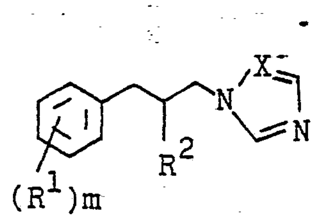

in der $R^1$ einen Alkylrest oder Halogen,
$R^2$ Wasserstoff, einen Alkylrest oder Aralkylrest, der durch Halogen substituiert sein kann,
m die Zahlen 0 bis 3 und
X CH oder N bedeuten,
sowie deren pflanzenphysiologisch verträgliche Säureadditionssalze und Metallkomplexe.

5. Verfahren zur Bekämpfung von Pilzen, dadurch gekennzeichnet, daß man die Pilze oder die vor Pilzbefall zu schützenden Gegenstände behandelt mit einem N-Phenylpropylsubstituierten Azol der Formel

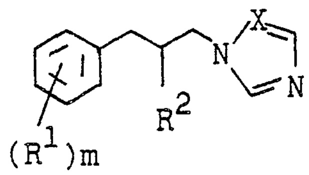

in der $R^1$ einen Alkylrest oder Halogen, $R^2$ Wasserstoff, einen Alkylrest oder Aralkylrest, der durch Halogen substituiert sein kann, m die Zahlen 0 bis 3 und X CH oder N bedeuten, sowie deren pflanzenphysiologisch verträgliche Säureadditionssalze und Metallkomplexe.

6. Verfahren zur Herstellung eines N-Phenylpropylsubstituierten Azol der Formel

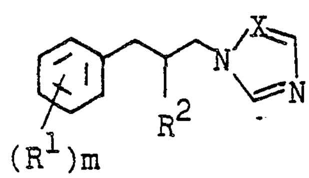

in der $R^1$ einen Alkylrest oder Halogen, $R^2$ Wasserstoff, einen Alkylrest oder Aralkylrest, der durch Halogen substituiert sein kann, m die Zahlen 0 bis 3 und X CH oder N bedeuten, sowie deren pflanzenphysiologisch verträgliche Säureadditionssalze und Metallkomplexe, dadurch gekennzeichnet, daß man ein Phenylpropylderivat der Formel

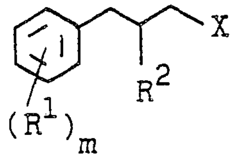

in der $R^1$, $R^2$ und m die obengenannten Bedeutungen haben und X Halogen oder einen Alkylsulfatrest oder p-Methyl-benzolsulfonyl oder p-Methoxybenzolsulfonyl bedeutet, mit 1,2,4-Triazol oder mit Imidazol bei einer Temperatur von 80 bis 200°C, gegebenenfalls in Gegenwart eines Säurefängers, umsetzt.

7. N-Phenylpropylsubstituiertes Azol ausgewählt aus der Gruppe bestehend aus 1-[3-(2,4-Dichlorphenyl)-2-n-propyl-propyl]-1,2,4-triazolium-nitrat,
1-[3-(2,4-Dichlorphenyl)-2-n-butyl-propyl]-1,2,4--triazolium-nitrat,
1-[3-(2,4-Dichlorphenyl)-2-n-butyl-propyl]-1,2,4-triazol,
N-[3-(2,4-Dichlorphenyl)-2-n-butyl-propyl]-imidazol,
1-[3-(2,4-Dichlorphenyl)-2-n-pentyl-propyl]-1,2,4--triazol
1-[3-(2,4-Dichlorphenyl)-2-benzyl]-1,2,4-triazol.

8. Fungizid enthaltend ein N-Phenylpropylsubstituiertes Azol ausgewählt aus der Gruppe bestehend aus 1-[3--(2,4-Dichlorphenyl)-2-n-propyl-propyl]-1,2,4-triazolium-nitrat,
1-[3-(2,4-Dichlorphenyl)-2-n-butyl-propyl]-1,2,4--triazolium-nitrat,
1-[3-(2,4-Dichlorphenyl)-2-n-butyl-propyl]-1,2,4-triazol,
N-[3-(2,4-Dichlorphenyl)-2-n-butyl-propyl]-imidazol,
1-[3-(2,4-Dichlorphenyl)-2-n-pentyl-propyl]-1,2,4--triazol
1-[3-(2,4-Dichlorphenyl)-2-benzyl]-1,2,4-triazol.

Patentansprüche für Österreich

1. Fungizid enthaltend N-Phenylpropylsubstituiertes Azol der Formel

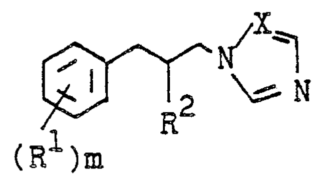

in der $R^1$ einen Alkylrest oder Halogen, $R^2$ Wasserstoff, einen Alkylrest oder Aralkylrest, der durch Halogen substituiert sein kann, m die Zahlen 0 bis 3 und X CH oder N bedeuten, sowie deren pflanzenphysiologisch verträgliche Säureadditionssalze und Metallkomplexe.

2. Fungizid enthaltend einen festen oder flüssigen Trägerstoff und ein N-Phenylpropylsubstituiertes Azol der Formel

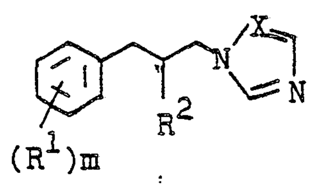

in der $R^1$ einen Alkylrest oder Halogen, $R^2$ Wasserstoff, einen Alkylrest oder Aralkylrest, der durch Halogen substituiert sein kann, m die Zahlen 0 bis 3 und X CH oder N bedeuten, sowie deren pflanzenphysiologisch verträgliche Säureadditionssalze und Metallkomplexe.

3. Verfahren zur Herstellung eines Fungizids, dadurch gekennzeichnet, daß man einen festen oder flüssigen Trägerstoff vermischt mit einem N-Phenylpropylsubstituierten Azol der Formel

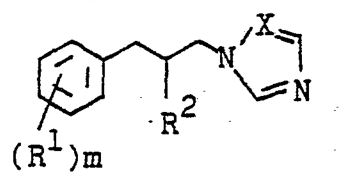

in der $R^1$ einen Alkylrest oder Halogen,
$R^2$ Wasserstoff, einen Alkylrest oder Aralkylrest, der durch Halogen substituiert sein kann,
m die Zahlen 0 bis 3 und
X CH oder N bedeuten,
sowie deren pflanzenphysiologisch verträgliche Säureadditionssalze und Metallkomplexe.

4. Verfahren zur Bekämpfung von Pilzen, dadurch gekennzeichnet, daß man die Pilze oder die vor Pilzbefall zu schützenden Gegenstände behandelt mit einem N-Phenylpropylsubstituierten Azol der Formel

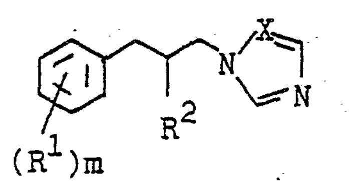

in der $R^1$ einen Alkylrest oder Halogen,
$R^2$ Wasserstoff, einen Alkylrest oder Aralkylrest, der durch Halogen substituiert sein kann,
m die Zahlen 0 bis 3 und
X CH oder N bedeuten,
sowie deren pflanzenphysiologisch verträgliche Säureadditionssalze und Metallkomplexe.

5. Verfahren zur Herstellung eines N-Phenylpropylsubstituierten Azol der Formel

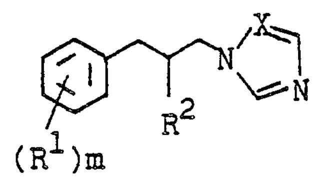

in der $R^1$ einen Alkylrest oder Halogen, $R^2$ Wasserstoff, einen Alkylrest oder Aralkylrest, der durch Halogen substituiert sein kann, m die Zahlen 0 bis 3 und X CH oder N bedeuten, sowie deren pflanzenphysiologisch verträgliche Säureadditionssalze und Metallkomplexe, dadurch gekennzeichnet, daß man ein Phenylpropylderivat der Formel

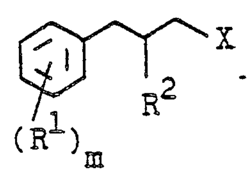

in der $R^1$, $R^2$ und m die obengenannten Bedeutungen haben und X Halogen oder einen Alkylsulfatrest oder p-Methyl-benzolsulfonyl oder p-Methoxybenzolsulfonyl bedeutet, mit 1,2,4-Triazol oder mit Imidazol bei einer Temperatur von 80 bis 200°C, gegebenenfalls in Gegenwart eines Säurefängers, umsetzt.

6. Fungizid enthaltend ein N-Phenylpropylsubstituiertes Azol ausgewählt aus der Gruppe bestehend aus 1-[3--(2,4-Dichlorphenyl)-2-n-propyl-propyl]-1,2,4-triazolium-nitrat,
1-[3-(2,4-Dichlorphenyl)-2-n-butyl-propyl]-1,2,4--triazolium-nitrat,
1-[3-(2,4-Dichlorphenyl)-2-n-butyl-propyl]-1,2,4-triazol,
N-[3-(2,4-Dichlorphenyl)-2-n-butyl-propyl]-imidazol,
1-[3-(2,4-Dichlorphenyl)-2-n-pentyl-propyl]-1,2,4--triazol
1-[3-(2,4-Dichlorphenyl)-2-benzyl]-1,2,4-triazol.

0025882

Europäisches Patentamt

# EUROPÄISCHER RECHERCHENBERICHT

Nummer der Anmeldung

EP 80 10 4975.0

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| X | US - A - 4 118 461 (ROHM AND HAAS CO.)<br>* Ansprüche 1 bis 5; Spalten 31 bis 32, Verbindung 67 *<br>-- | 1-3, 5-8 | C 07 D 249/08<br>C 07 D 233/56<br>C 07 D 233/58<br>A 01 N 43/64<br>A 01 N 43/50 |
| X | EP - A1 - 0 001 809 (BASF)<br>* Anspruch 1; Verbindung Seite 6 *<br>-- | 1,7 | |
| X | EP - A1 - 0 003 211 (BASF)<br>* Seite 5, Verbindungen 6 und 8 *<br>-- | 1 | RECHERCHIERTE SACHGEBIETE (Int. Cl.³) |
| | US - A - 3 927 017 (JANSSEN PHARMACEU-TICA)<br>* Beispiel XXXV, Spalte 3, Zeilen 64 bis 68 *<br>-- | 1,2 | A 01 N 43/50<br>A 01 N 43/64<br>C 07 D 233/56<br>C 07 D 233/58<br>C 07 D 249/08 |
| P | EP - A1 - 0 008 651 (BASF)<br>* Anspruch 1 *<br>-- | 1 | |
| D | DE - A1 - 2 656 747 (BASF)<br>* Anspruch 2 *<br>---- | 2 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt. | | |

| Recherchenort | Abschlußdatum der Recherche | Prüfer |
|---|---|---|
| Berlin | 18-12-1980 | FROELICH |